# EUROPEAN PATENT APPLICATION

(11) **EP 4 613 326 A1**
(43) Date of publication of application: **10.09.2025**
(21) Application number: 23897517.1
(22) Date of filing: 16.11.2023
(51) Int. Cl.: A61N 5/10, H01J 27/24

(54) **ION GENERATION DEVICE, ION GENERATION METHOD, AND TARGET FOR ION GENERATION**

(30) Priority: 29.11.2022 JP 2022190747
(71) Applicant: National Institutes for Quantum Science and Technology, Chiba 263-8555 (JP); Kanadevia Corporation, Osaka-shi, Osaka 559-8559 (JP)
(72) Inventor: KOJIMA, Sadaoki, Chiba-shi, Chiba 263-8555 (JP); SAKAKI, Hironao, Chiba-shi, Chiba 263-8555 (JP); MIYATAKE, Tatsuhiko, Chiba-shi, Chiba 263-8555 (JP); KONDO, Kiminori, Chiba-shi, Chiba 263-8555 (JP); KUROKI, Hiroyoshi, Osaka-shi, Osaka 559-8559 (JP); SHIMIZU, Yusuke, Osaka-shi, Osaka 559-8559 (JP); HARADA, Hisanori, Osaka-shi, Osaka 559-8559 (JP); INOUE, Norihiro, Osaka-shi, Osaka 559-8559 (JP)
(74) Representative: Patentanwaltskanzlei WILHELM & BECK
(86) International application number: PCT/JP2023/041213
(87) International publication number: WO 2024/116866

(57) **Abstract**

Provided is an ion generation device that makes it possible to sequentially supply a target to an irradiation region while removing an impurity layer which can be formed on a surface of the target. An ion generation device (10) includes: an induction heating section (13) that carries out induction heating with respect to a target (TA) which includes a conductor layer and which is in film form; and a laser irradiation section (laser light source 15, focusing mirror 16b) that irradiates the target with laser light so as to generate ions from the target, the target having been subjected to induction heating.

## Description

### Technical Field

The present invention relates to an ion generation device, an ion generation method, and a target for ion generation.

### Background Art

An ion generation device that generates and emits desired ions is used in, for example, cancer therapy.

Non-patent Literature 1 discloses an ion generation device that generates desired ions (carbon ions) by irradiating, with laser light, a film (target) which is made of carbon and which is a source of the desired ions. In a case where a surface (first surface) of the target is irradiated with high-power laser light, a large number of high energy electrons are ejected from a surface (second surface) on a side facing away from the surface, which is irradiated with the high-power laser light laser light. This results in generation of an intense sheath electric field on or near the second surface, so that carbon ions are ejected from the target by the sheath electric field and are emitted as desired ions.

### Citation List

### [Non-patent Literature]

[Non-patent Literature 1]
   L. Torrisi et. al., Physical Review Accelerators Beams., 23, 011304 (2020).
[Non-patent Literature 2]
   M. Hegelich et. al., PHYSICAL REVIEW LETTERS, 89, 085002 (2002).

### Summary of Invention

### Technical Problem

This ion generation device may generate impurity ions (e.g., hydrogen ions) other than the desired ions (e.g., carbon ions). This is because an impurity layer (water molecule layer) on which impurities such as moisture are adsorbed is formed on a surface of the target. Generation of ions by irradiating the target with laser light after removing the impurity layer from the target is considered in order to prevent generation of impurity ions.

A method for removing the impurity layer from the target is, for example, a resistance heating method in which a large electric current is allowed to pass through the target and energy of Joule heat caused by such an electric current is used. In order to heat the target with use of the resistance heating method, a pair of electrodes for allowing a large electric current to pass through the target is formed on the target, and a power source is connected to the pair of electrodes.

In a case where an ion generation device is used in, for example, cancer therapy, there is a request that high intensity ions should be generated from the ion generation device at a high frequency. To this end, it is preferable to sequentially supply, to an irradiation region that is irradiated with laser light, a target from which an impurity layer has been removed by heating. However, in the foregoing resistance heating method, the power source is connected to the pair of electrodes that are formed on the target. This makes it difficult to sequentially supply the target to the irradiation region.

An aspect of the present invention has been made in view of the foregoing problem, and an object thereof is to provide an ion generation device that makes it possible to sequentially supply a target to an irradiation region while removing an impurity layer which can be formed on a surface of the target.

### Solution to Problem

In order to attain the object, an ion generation device in accordance with an aspect of the present invention includes: an induction heating section that carries out induction heating with respect to a target which includes a conductor layer and which is in film form; and a laser irradiation section that irradiates the target with laser light so as to generate ions from the target, the target having been subjected to induction heating.

In order to attain the object, an ion generation method in accordance with an aspect of the present invention includes: the step of carrying out induction heating with respect to a target that includes a conductor layer and that is in film form; and the step of irradiating the target with laser light so as to generate ions from the target, the target having been subjected to induction heating.

In order to attain the object, a target for ion generation in accordance with an aspect of the present invention includes: a conductor layer; and a first ion source layer that is provided on at least one main surface of the conductor layer and that is composed of a first ion species which is ionized by being irradiated with laser light.

### Advantageous Effects of Invention

An aspect of the present invention makes it possible to provide an ion generation device that makes it possible to sequentially supply a target to an irradiation region while removing an impurity layer which can be formed on a surface of the target.

### Brief Description of Drawings

Fig. 1 is a diagram schematically illustrating an ion generation device in accordance with Embodiment 1.
Fig. 2 is a diagram representing a specific example of an induction heating section.
Fig. 3 is a diagram representing a cross section of a target.
Fig. 4 is a diagram representing ions emitted from a target.
Fig. 5 is a diagram representing a movement section of an ion generation device in accordance with Embodiment 2.
Fig. 6 is a diagram representing a temperature distribution of a target heated by an induction heating section.
Fig. 7 provides graphs each representing a change in temperature of a target over time.
Fig. 8 shows a result of analysis of ions emitted from a target consisting of a single layer of nickel.
Fig. 9 provides graphs representing energy spectra of ions emitted from a target consisting of a single layer of nickel.
Fig. 10 is a diagram schematically illustrating a pollutant layer having adhered to a structure of a target TA which structure is assumed from a result of detection of ions.
Fig. 11 shows a result of analysis of ions emitted from a target consisting of two layers of nickel and carbon.
Fig. 12 provides graphs representing energy spectra of ions emitted from a target consisting of two layers of nickel and carbon.
Fig. 13 is a diagram schematically illustrating a structure of a target which structure is assumed when a target consisting of three layers of nickel, argon, and carbon is placed in the atmosphere.
Fig. 14 provides graphs representing ions emitted from a target consisting of three layers of nickel, argon, and carbon.

### Description of Embodiments

### [Embodiment 1]

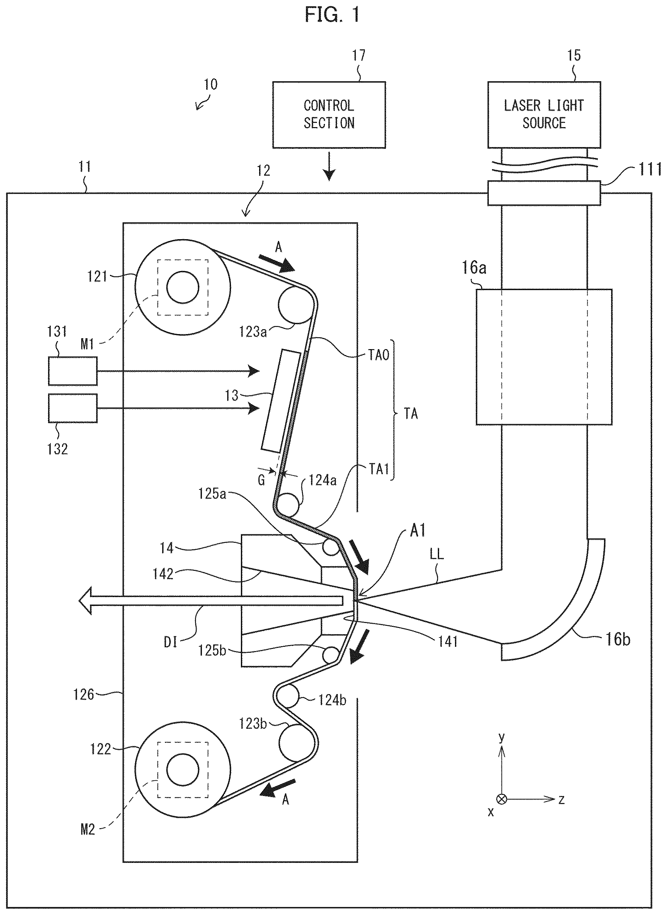

The following description will discuss, with reference to Fig. 1, an ion generation device 10 in accordance with Embodiment 1 of the present invention. Fig. 1 is a diagram schematically illustrating the ion generation device 10 in accordance with Embodiment 1.

### <Ion generation device>

As illustrated in Fig. 1, the ion generation device 10 includes a chamber 11, a movement section 12, an induction heating section 13, a holding section 14, a laser light source 15, a pulse compressor 16a, a focusing mirror 16b, and a control section 17.

The ion generation device 10 can generate various ions, e.g., carbon ions, ions of metals (such as nickel), and ions of gasses (such as helium, argon, oxygen, and neon), and has various applications. Such an application is, for example, an application to a heavy ion cancer therapy device (commonly referred to as a quantum scalpel). In a laser-driven ion injector mounted on a heavy ion cancer therapy device, the ion generation device 10 can be used to generate high purity heavy ions.

### (Chamber)

The chamber 11 is a container that is made of metal (stainless steel in the present embodiment). In Fig. 1, a single solid line is used to simply illustrate a shape of the chamber 11. Note, however, that the chamber 11 actually has a thickness which is set as appropriate.

The chamber 11 is configured so as to be able to close an internal space thereof. To the chamber 11, a vacuum pump (not illustrated in Fig. 1) is connected. The vacuum pump keeps a pressure in the internal space lower than an atmospheric pressure by evacuating the internal space of the chamber 11. In the present embodiment, the pressure in the internal space of the chamber 11 is approximately 1×10⁻² Pa. Note, however, that the pressure in the internal space of the chamber 11 is not limited to the above pressure and can be set as appropriate.

The chamber 11 has a port 111. The port 111 is a light input/output port and is made of a plate-like member that is made of glass which is quartz glass and that allows laser light LL (described later) to be transmitted therethrough. As described later, the laser light LL has a center wavelength of, for example, 810 nm. Note, however, that a material of which the port 111 is made is not limited to quartz glass and may be any material that is light-transmissive from a visible region to an infrared region. In the following description, the center wavelength of the laser light LL is also simply referred to as a wavelength of the laser light LL.

### (Movement section)

The movement section 12 moves a target TA and supplies the target to the induction heating section 13 and the holding section 14. The movement section 12 moves the target TA from pulleys 123a and 124a (a first holding section) to the holding section 14 (a second holding section). That is, the first holding section and the second holding section are spaced apart from each other when an irradiation region irradiated with the laser light LL is viewed in a plan view.

The target TA is a film-like material at least some of components of which are electrically conductive and which contains an ion species. In a case where the target TA is irradiated with the laser light LL from the laser light source 15, the ion species is ionized and emitted as laser-driven ions DI from the target TA. This will be described in detail later.

The movement section 12 includes pulleys 121, 122, 123a, 123b, 124a, 124b, 125a, and 125b, motors M1 and M2, and a base material 126. In Embodiment 1, the target TA is in tape form having a long length with respect to a width and is continuously moved by the pulleys 121, 122, etc., so that the laser-driven ions DI can be continuously generated.

The pulley 121 includes a rotating shaft that is configured to be rotatable. To the rotating shaft, a core (hereinafter referred to as a first core) is fixed on which one end of the target TA is wound. Thus, the first core rotate together with the pulley 121. The pulley 121 functions as a first pulley through which the target TA is fed.

In the present embodiment, the other end of the target TA is fixed to a second core that is a hollow cylinder.

As in the case of the pulley 121, the pulley 122 includes a rotating shaft that is configured to be rotatable. To the rotating shaft, the second core is fixed on which the other end of the target TA is wound. Thus, the second core can rotate together with the pulley 121. The pulley 122 functions as a second pulley around which the target TA is wound.

The pulleys 123a, 123b, 124a, 124b, 125a, and 125b are provided between the pulley 121 and the pulley 122 and define a path for the target TA from the 121 to the pulley 122. As in the case of the pulleys 121 and 122, the pulleys 123a, 123b, 124a, 124b, 125a, and 125b also include respective rotating shafts each of which is configured to be rotatable.

The pulleys 121, 122, 123a, 123b, 124a, 124b, 125a, and 125b allow the target TA to be continuously fed along arrows A from the pulley 121 to the pulley 122. Thus, the pulley 121 is an example of the first pulley through which the target TA is fed, and the pulley 122 is an example of the second pulley around which the target TA is wound. Note that arrangement and the number of the pulleys 123a, 123b, 124a, 124b, 125a, and 125b can be changed as appropriate.

The motors M1 and M2 are, for example, stepping motors. The motor M1 includes a rotating shaft that is configured to be rotatable. The rotating shaft of the motor M1 is mechanically coupled to the rotating shaft of the pulley 121. The motor M2 has a configuration similar to the configuration of the motor M1, and a rotating shaft of the motor M2 is mechanically coupled to the rotating shaft of the pulley 122. Thus, rotation of the rotating shafts of the motors M1 and M2 drives the respective pulleys 121 and 122.

The base material 126 is a plate-like member which is made of metal (for example, stainless steel) and a pair of main surfaces of which has a rectangular shape. The pulleys 121, 122, 123a, 123b, 124a, 124b, 125a, and 125b and the motors M1 and M2 are provided on one of the main surfaces of the base material 126. Although not illustrated in Fig. 1, a stage that makes it possible to translate a position of the base material 126 at least in a z-axis direction is preferably provided below the base material 126.

The target TA is heated by the induction heating section 13 as described later. Thus, the target TA may be deformed by thermal expansion, thermal strain, or the like, and a position thereof may be displaced from a predetermined laser irradiation position. Note that the target TA in the present embodiment is in tape form. This prevents the target TA from deviating from the predetermined laser irradiation position even in a case where deformation occurs in an x-axis direction and a y-axis direction. Deformation in the target TA which deformation causes concern here is deformation in the z-axis direction (deformation such that the target TA which is in tape form waves). In preparation for a case where such deformation in the z-axis direction occurs, the ion generation device 10 preferably has a positional displacement correction mechanism that measures positional displacement of the target TA in the z-axis direction and that subjects the base material 126 to parallel translation so as to solve such a positional displacement problem.

The positional displacement correction mechanism includes, for example, a positional displacement detector that detects positional displacement of the target TA and a movement mechanism that causes a coincidence between a position of the target TA in the z-axis direction and a position of the laser irradiation position in the z-axis direction on the basis of a detection result from the positional displacement detector. The positional displacement detector can be, for example, a surface displacement meter. The movement mechanism may be configured to cause a coincidence between the position of the target TA in the z-axis direction and the position of the laser irradiation position in the z-axis direction by finely adjusting the holding section 14 in the z-axis direction. Alternatively, the movement mechanism may be configured to cause a coincidence between the position of the target TA in the z-axis direction and the position of the laser irradiation position in the z-axis direction by finely adjusting a focus of the laser light LL. That is, the movement mechanism may be configured to move a position of the focus of the laser light LL in the z-axis direction.

### (Induction heating section)

The induction heating section 13 heats the target TA to remove an impurity layer of, for example, moisture adhering to the target TA and prevents ions different from desired ions, such as hydrogen ions (protons) from being released from the target TA. The induction heating section 13 functions as an induction heating section that carries out induction heating with respect to the target TA which includes a conductor layer and which is in film form.

In a case where an object existing in nature is exposed to the atmosphere, impurities such as moisture adhere to a surface of the object, and an impurity layer such as a water molecule layer is formed. Even in a case where the target TA that is clean is prepared, a water molecule is spontaneously adsorbed on a surface of the target TA either in the atmosphere or in a vacuum of approximately 0.01 Pa. Thus, in order to use the target TA, it is necessary to remove surface impurities in advance.

A water molecule that has physically adsorbed on the target TA can be removed by evacuation. In contrast, a water molecule that has chemically adsorbed on the target TA is difficult to remove by evacuation but can be desorbed by heating the target TA.

Note here that it is preferable to continuously supply the target TA in order to continuously generate ions. In this case, it is preferable to remove the impurity layer by rapidly (for example, in a time of approximately 1 second or less) causing the target TA reach a temperature of several hundred degrees centigrade.

The induction heating section 13 is a heating mechanism that heats the target TA by applying a high frequency magnetic field to the target TA, for example, an induction heating coil that is close to the target TA. As described earlier, at least a part of the target TA is electrically conductive. Thus, application of a temporally fluctuating magnetic field to the target TA induces an induced eddy current in the target TA. This induced eddy current is converted into heat by internal resistance of the target TA, so that the target TA generates heat.

In this case, a part of the target TA which part is heated by the induction heating section 13 is held in a planar manner by the pulleys 123a and 124a and faces the induction heating section 13 with a gap G between the part and the induction heating section 13. That is, the pulleys 123a and 124a function as the first holding section that holds the target TA so as to bring the target TA close to the induction heating section. The pulleys 123a and 124a and the holding section 14 function as a holding section that holds the target so as to bring the target close to the induction heating section.

To the induction heating section 13, a power source 131 and a cooler 132 are connected. The power source 131 supplies an AC voltage to the induction heating section 13 to generate a fluctuating magnetic field. The cooler 132 prevents the induction heating section 13 itself from being excessively heated and damaged by, for example, radiant heat from the target TA that has generated heat.

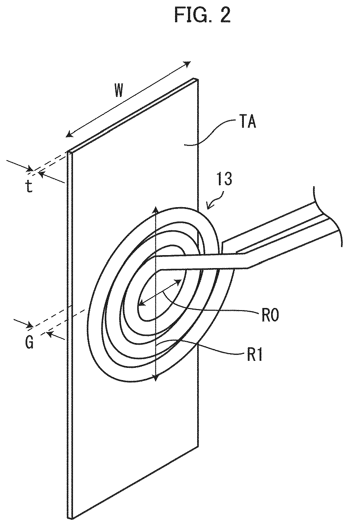

Fig. 2 is a diagram representing a specific example of the induction heating section 13. The induction heating section 13 is an induction heating coil that is composed of a conductor pipe wound in a spiral manner. Supply of, for example, an AC voltage of 400 kHz from the power source 131 to the conductor pipe (induction heating coil) generates a fluctuating magnetic field. The induction heating section 13 can be cooled by causing cooling water to flow through the pipe. That is, in the present embodiment, the cooler 132 is a refrigerant supply device that supplies a refrigerant to the induction heating section 13. Note, however, that the refrigerant may be not only a liquid such as cooling water but also a gas.

In the present embodiment, a copper pipe that is wound three times is used as the conductor pipe. However, a shape of a coil, the number of times the coil is wound, and a material of the coil can be changed as appropriate.

The induction heating section 13 is provided substantially parallel to the target TA at a place that is separated by the gap G (4 mm in the present embodiment) from the target TA. The induction heating section 13 heats a target TAO that has been conveyed and that is in a non-heated state, so that the non-heated target TAO is made into a target TA1 that is in a heated state.

Heating by the induction heating section 13 has the following advantages as compared with other heating means.
(1) A heating speed is high. Thus, by supplying the target TA at a high speed and sequentially heating the target TA, it is possible to continuously generate the laser-driven ions DI that have high intensity.
(2) The target TA can be heated in a noncontact manner. Thus, it is unnecessary to mount an electrode on the target TA, and it is easy to replace the target TA.
(3) A region in which the target TA is heated is limited to a region to which a magnetic field is applied. Thus, influence of heat from the induction heating section 13 on surroundings of the induction heating section 13 is limited.
(4) As compared with a laser ablation method (in which a pulse laser having an intensity exceeding an ablation threshold is used), an induction heating method makes it less likely that unnecessary gas will be produced, so that less load is imposed on an evacuation system.

### (Holding section)

The holding section 14 is a block-like member that is made of metal (for example, stainless steel). When viewed in a direction normal to the main surfaces of the base material 126, the holding section 14 is formed in a shape of a decagon obtained by combining two large and small hexagons (see Fig. 1). A pair of surfaces of the holding section 14 which surfaces are substantially parallel to the main surfaces of the base material 126, the holding section 14 being formed in the shape of a decagon as described above, is referred to as a pair of main surfaces, and surfaces of the holding section 14 which surfaces constitute contours of the pair of main surfaces are referred to as outer surfaces.

Note that, as long as the holding section 14 basically holds the target TA in the irradiation region irradiated with the laser light LL and ions generated by emission of the laser light LL can be extracted, an appropriate shape different from the shape in the present embodiment can be selected, as appropriate, as the shape of the holding section 14.

The holding section 14 is located between the pulley 121 and the pulley 122 when viewed along the path for the target TA. More specifically, the holding section 14 is provided so that a smaller hexagon of the two large and small hexagons (described earlier) is positioned between the pulley 125a and the pulley 125b, and a part of the smaller hexagon protrudes from the negative z-axis direction side circumscribed surface among circumscribed surfaces which are circumscribed about the pulleys 125a and 125b. Thus, a head surface 141, which is at least the negative z-axis direction side end surface among the outer surfaces of the holding section 14, comes into contact with the target TA that is extruded from the negative z-axis direction side circumscribed surface (described above) in the negative z-axis direction.

The holding section 14 may be magnetic (preferably ferromagnetic). Further, in a case where the holding section 14 is magnetic, the conductor layer included in the target TA is also preferably magnetic (preferably ferromagnetic). Moreover, magnetism of the holding section 14 and magnetism of the conductor layer of the target TA are preferably set so that an attractive force is exerted between the head surface 141 and the target TA. As described earlier, positional displacement from the predetermined laser irradiation position in the z-axis direction may occur in the target TA due to the target TA being heated. According to the above configuration, an attractive force is exerted between the head surface 141 and the target TA. This allows the target TA to adhere to the head surface 141. Thus, it is possible to prevent or reduce positional displacement that can occur in the z-axis direction of the target TA. An adhesion mechanism for causing the target TA to adhere to the head surface 141 may be used together with the foregoing positional displacement correction mechanism, or either the adhesion mechanism or the positional displacement correction mechanism may be used.

Note that the holding section 14 can adjust its position in a direction of an arrow B which direction is parallel to the z-axis direction. Thus, the holding section 14 can arbitrarily adjust an amount in which the head surface 141 protrudes from the negative z-axis direction side circumscribed surface (described earlier). In other words, the holding section 14 can use the head surface 141 to determine the position in a direction normal to the main surfaces of the target TA (in the z-axis direction in Fig. 1). The head surface 141 holds the target TA in a planar manner at a beam spot A1. That is, the holding section 14 functions as the second holding section that holds the target TA in the irradiation region irradiated with the laser light LL.

A groove 142 is formed on a main surface (negative x-axis direction side main surface) of the pair of main surfaces of the holding section 14 which main surface is farther from the base material 126. The groove 142 has a trapezoidal shape when viewed in the direction normal to the main surfaces of the base material 126. A pair of bases of the groove 142 are located on the positive z-axis direction side edge and the negative z-axis direction side edge, respectively, of the contours of the holding section 14. That is, the positive z-axis direction side end surface and the head surface 141 among the outer surfaces of the holding section 14 are provided with respective notches, which are connected by the groove 142.

### (Laser light source)

The laser light source 15 emits the laser light LL. As described later, the focusing mirror 16b is used to irradiate the target TA with the laser light LL that has been emitted from the laser light source 15, and ions (e.g., C⁴⁺) are generated from the target TA. The laser light source 15 and the focusing mirror 16b function as a laser irradiation section that irradiates the target TA with laser light so as to generate ions from the target TA, the target TA having been subjected to induction heating.

The laser light source 15 employed in the present embodiment is a Ti:sapphire laser that emits the laser light LL which has a wavelength of 810 nm. In the present embodiment, the laser light LL that enters the focusing mirror 16b is collimated light which has a wavelength of 810 nm, a pulse width of 80 fs, and energy per pulse of approximately 500 mJ. The laser light source 15 and an optical axis of the laser light LL are set so that the beam spot A1 (described later) has a diameter of not less than 2 µm and not more than 3 µm. Note, however, that a wavelength and energy per pulse of the laser light LL change kinetic energy of ions generated from the target TA. Thus, the wavelength and the energy per pulse of the laser light LL can be selected as appropriate in accordance with required energy.

In the present example embodiment, the target TA is heated by the induction heating section 13 so that the target TA from which an impurity layer of, for example, moisture has been removed is irradiated with the laser light LL. That is, heating of the target TA and emission of the laser light LL are sequentially carried out. This configuration makes it easy to sufficiently heat the target TA. Note, however, that a travel time, which is a time from heating of the target TA until the target TA is irradiated with the laser light LL, is preferably as short as possible. The travel time is preferably not more than 5 seconds in a case where the pressure in the internal space of the chamber 11 is approximately 1×10⁻² Pa. A longer travel time results in readsorption of the impurity layer and a decrease in effect (i.e., removal of the impurity layer) due to heating of the target TA. Thus, the longer travel time reduces the number and maximum energy of generated ions and increases the number and maximum energy of generated hydrogen ions (H⁺).

The ion generation device 10 carries out an ion generation method including: the step of carrying out induction heating with respect to the target TA that includes the conductor layer and that is in film form; and the step of irradiating the target TA with the laser light LL so as to generate ions from the target TA, the target TA having been subjected to induction heating. In an embodiment, these steps (heating of the target TA and emission of the laser light LL) are concurrently carried out. That is, while being subjected to induction heating by the induction heating section 13, the target TA uses the laser light LL that has been emitted from the laser irradiation section to generates ions.

In the present embodiment, heating of the target TA and emission of the laser light LL are carried out in different regions of the target TA. In contrast, these regions may be overlapped or identical. For example, the laser light LL is emitted through a central space of an induction coil constituting the induction heating section 13. In this case, when the irradiation region irradiated with the laser light LL is viewed in a plan view, the irradiation region is included in a region of the target which region is close to the induction heating section 13. This makes it possible to prevent the effect due to heating of the target TA from being reduced in accordance with the travel time.

The laser light source 15 is disposed so that the laser light LL enters the internal space of the chamber 11 through the port 111.

The pulse compressor 16a and the focusing mirror 16b are provided in this order on the optical axis of the laser light LL in the internal space of the chamber 11. The pulse compressor 16a compresses the laser light LL in a time direction to, for example, 80 fs. By reflecting the laser light LL while converting, into convergent light, the laser light LL that is collimated light, the focusing mirror 16b irradiates, with the laser light LL that is convergent light, the target TA that is held in a planar manner by the holding section 14. The focusing mirror 16b employed in the present embodiment is an off-axis parabolic mirror. Thus, the beam spot A1 that is a part of the main surfaces of the target TA is irradiated, via the focusing mirror 16b, with the laser light LL which has entered the internal space of the chamber 11 through the port 111.

The laser light LL may be continuously or intermittently emitted. Note that, since the laser light source 15 generates a pulse laser, it is possible to consider that continuous emission of the laser light LL means, for example, emission of a pulse laser at approximately 100 Hz and that intermittent emission means, for example, emission of a pulse laser in a cycle which is sufficiently smaller than 100 Hz (e.g., a single shot).

### (Control section)

The control section 17 controls the movement section 12, the induction heating section 13, and the laser light source 15. The control section 17 drives the movement section 12 by controlling the motors M1 and M2, and conveys the target TA from the pulley 121 to the induction heating section 13, the holding section 14, and the pulley 122 in this order.

### (Target)

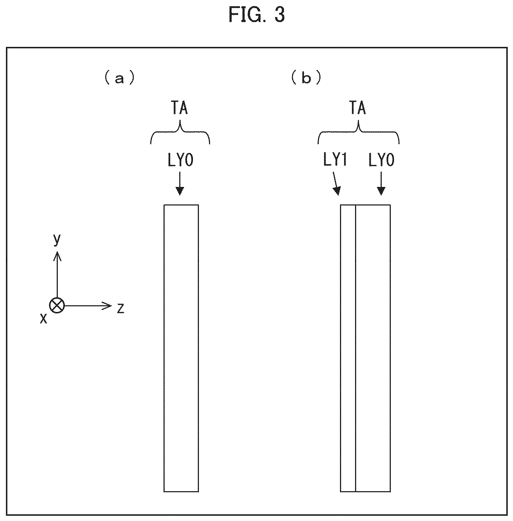

Fig. 3 is a diagram representing a cross section of the target TA. The target TA, which is a target for ion generation and is in film form, may be composed of either a single layer LYO as illustrated in (a) of Fig. 3 or a plurality of layers LYO and LY1 as illustrated in (b) of Fig. 3. Although (b) of Fig. 3 illustrates the target TA consisting of two layers, the target TA may consist of three layers or more.

In the present embodiment, the target TA is formed in the form of a tape. The target TA has one end that is fixed to a core which is a hollow cylinder. The target TA the one end of which is fixed to the core is wound on the core. The target TA has a larger width W than a region that is irradiated with the laser light LL.

A part of the target TA which part faces the induction heating section 13 is held in a planar manner by the pulleys 123a and 124a (first holding section), and a part of the target TA which part is irradiated with the laser light LL is held in a planar manner by the holding section 14 (second holding section). A mechanism by which the first and second holding sections hold the target TA is not limited and can be selected as appropriate.

A conductor (for example, a metal film, e.g., a nickel film) can be used as the layer LYO of the target TA. Using the conductor as the layer LYO makes it possible to (i) cause the induction heating section 13 to carry out induction heating with respect to the target TA and (ii) remove the impurity layer adhering to the target TA.

In a case where the target TA consists of a single layer, irradiating the target TA with the laser light LL makes it possible to ionize atoms constituting the conductor layer LYO and emit the ionized atoms as the laser-driven ions DI. This makes it possible to accelerate heavy ions having a wide range of atomic numbers with electroconductivity. For example, in a case where the conductor is made of nickel, nickel ions are generated. In such a case, the target TA has the conductor layer LYO that is composed of an ion species which is ionized by being irradiated with the laser light LL.

The following description will discuss a case where the target TA has the conductor layer LYO and the ion species which is ionized by being irradiated with the laser light LL. In this case, (1) the layer LY1 containing the ion species may be formed on the conductor layer LYO, or (2) the ion species may be embedded in the conductor layer LYO. The present embodiment assumes that the ion species excludes hydrogen ions. That is, the ion species has atoms having two or more atoms.

In a case where the layer LY1 containing the ion species is formed on the conductor layer LYO, the target TA may include: the conductor layer LYO; and the first ion source layer LY1 that is provided on at least one main surface of the conductor layer LYO and that is composed of a first ion species which is ionized by being irradiated with the laser light. That is, one layer LY1 containing the ion species may be disposed on one main surface of the conductor layer LYO, and another layer containing the ion species may be disposed on the other main surface of the conductor layer LYO. Further, the target TA may further include a second ion source layer that is formed at or near an interface of the conductor layer LYO with the first ion source layer LY1 and that contains a second ion species which is ionized by being irradiated with laser light.

Such a target TA can be manufactured by a manufacturing method including: the step of preparing a conductor film (the conductor layer LYO); and the step of embedding, in the conductor film, an ion species that is ionized by being irradiated with the laser light LL, or forming, on the conductor film, a layer containing an ion species. In this case, the step of embedding, in the conductor film, the ion species that is ionized by being irradiated with the laser light can include the step of subjecting the conductor film to ion sputtering of the ion species. Ion sputtering will be described later.

For example, the target TA may have a multilayer structure consisting of the conductor layer LYO and the layer LY1 containing the ion species. The conductor layer LYO allows the induction heating section 13 to carry out induction heating with respect to the target TA, and emission of the laser light LL allows the ion species contained in the layer LY1 to be ionized and emitted as the laser-driven ions DI. This makes it easy to use, as the ion species, not only a conductor material but also a nonconductor material and a material that is gaseous at normal temperature. Note that the layer LY1 may be made of either a conductor material or a nonconductor material. As described later, the layer LY1 is preferably formed on a back surface F2 on a side facing away from a front surface F1 (surface irradiated with the laser light LL) of the conductor layer LYO. It is possible to prevent a material of which the conductor layer LYO is made from being ionized and emitted.

The layer LY1 can be made of, for example, carbon (C) to generate and emit carbon ions (e.g., C⁴⁺).

The layer LY1 can be a layer of a substance (e.g., helium, argon, oxygen, or neon) that is gaseous at normal temperature. The layer LY1 can be formed by ionizing and accelerating gas and causing the gas to collide with and adhere to the conductor layer LYO. For this, an ion sputtering method can be used.

However, in this case, depending on a preparation condition, ionized gas atoms sometimes do not form a distinct layer on the conductor layer LYO. For example, ions that are accelerated at a high speed can be embedded in the conductor layer LYO and can be dispersed and disposed as gas atoms (or molecules) in the conductor layer LYO.

In the present embodiment, the conductor layer LYO has a thickness of 5 µm. The thickness of the layer LY1 containing the ion species varies depending on the ion species, but can be, for example, 0.1 µm in a case where the ion species is carbon (C). That is, in the present embodiment, the target TA has a thickness of approximately 5 pm.

The thickness of the target TA is preferably not less than 100 nm and not more than 12.5 µm. Further, the thickness of the target TA is more preferably not less than 1 µm and not more than 5 µm. The target TA that has a smaller thickness allows ions generated from the target TA to have higher acceleration energy.

### (Principle of ion generation)

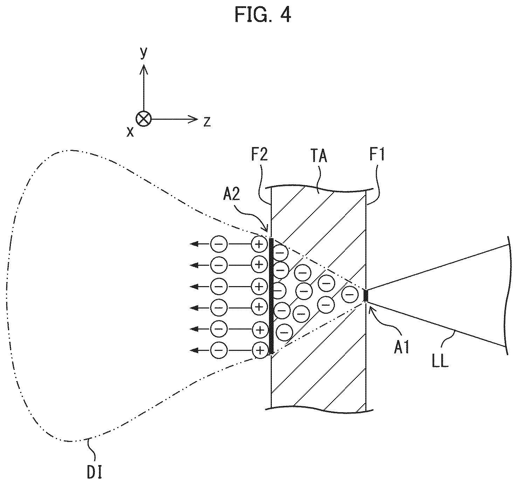

Fig. 4 is a diagram representing ions emitted from the target TA. As illustrated in Fig. 4, in a case where the region (beam spot) A1 included in the front surface F1 of the target TA is irradiated with the laser light LL, electrons that are present at or near the beam spot A1 vigorously vibrate due to an interaction occurring between the electrons and the laser light LL, and are accelerated in a direction (positive z-axis direction in Fig. 4) from the front surface F1 toward the back surface F2 (forward acceleration), and are ejected from an ion generation region A2 of the back surface F2 to outside the target TA. In this case, the electrons that have been ejected from the back surface F2 to outside the target TA generate a sheath electric field between the electrons and ions remaining in the target TA. Note that Fig. 4 uses an imaginary line (two-dot chain line) to schematically illustrate a range of a region in which the electrons propagate inside the target TA while diverging.

The ions remaining in the target TA are accelerated by the sheath electric field (backward acceleration, i.e., a target normal sheath acceleration mechanism) and ejected from the back surface F2 to outside the target TA. Note that Fig. 4 uses an imaginary line (two-dot chain line) to schematically illustrate a shape of a region in which the ejected ions are distributed in a space that is located on the back surface F2 side.

The ions that are ejected, as described above, from the back surface F2, which is the main surface on a side facing away from the front surface F1 that has been irradiated with the laser light LL, are referred to as backward-accelerated ion ions. Furthermore, as disclosed in Phys. Rev. Lett. 99, 185002 (2007), it is known that ions are ejected also from the front surface F1 in a case where the front surface F1 is irradiated with the laser light LL in which the ratio of background light (prepulses) to main pulses is small. The ions that are thus ejected from the front surface F1 are referred to as backward-accelerated ions. Ions to be emitted can be either forward-accelerated ions or backward-accelerated ions.

Note here that the sheath electric field has a property of selectively accelerating ions having a large mass-to-charge ratio. It is therefore preferable to remove an impurity layer (water molecule layer) adhering to the target TA. Hydrogen ions (protons) in which hydrogen contained in the impurity layer (water molecule layer) has been electrolytically dissociated have a mass-to-charge ratio (electric charge amount/mass number) of 1, which is larger than that of other ions (whose mass-to-charge ratio is at most 1/2). Thus, in a case where hydrogen ions are present, most of energy possessed by the sheath electric field is used to accelerate the hydrogen ions. This significantly reduces efficiency with which the other ions are accelerated.

### <Control section>

The control section 17 controls the motors M1 and M2 as described earlier. The control section 17 also controls the laser light source 15 that emits the laser light LL.

A function of the control section 17 can be realized by a program for causing a computer to function as the control section 17. In this case, the control section 17 includes, as hardware for executing the program, a computer that has at least one control device (e.g., a processor) and at least one storage device (e.g., a memory). Using the at least one control device and the at least one storage device to execute the program allows the control section 17 to control the motors M1 and M2 and the laser light source 15.

### [Embodiment 2]

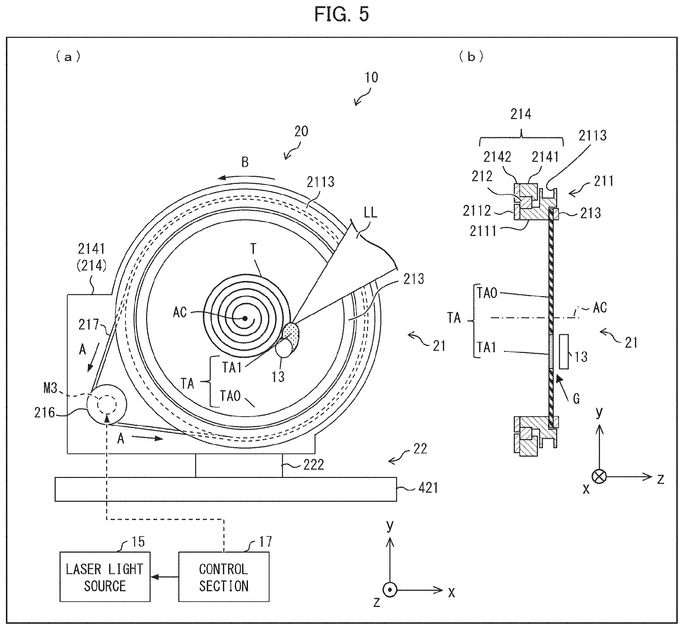

The following description will discuss, an ion generation device 10 in accordance with Embodiment 2 of the present invention. Fig. 5 is a diagram representing a movement section 20 of an ion generation device in accordance with Embodiment 2. That is, the ion generation device 10 in accordance with Embodiment 2 includes the movement section 20 instead of the movement section 12 of Embodiment 1.

(a) of Fig. 5 is a plan view of the movement section 20. (b) of Fig. 5 is a cross-sectional view of a rotational movement stage 21 included in the movement section 20. Note that for convenience, members having functions identical to those of the respective members described in Embodiment 1 are given respective identical reference numerals, and a description of those members is omitted.

The movement section 12 of Embodiment 1 continuously feeds the target TA in a longer direction of the target TA, the target TA having been formed in the form of a tape. In contrast, the movement section 20 of Embodiment 2 continuously feeds a target TA by moving the target TA in a plane of a main surface of the target TA (in a plane parallel to an xy plane in (a) of Fig. 5), the target TA having been formed in a disk shape, for example, in a circular shape or a polygonal shape.

### <Target TA>

The target TA of Embodiment 2 is formed in a disk shape, for example, in a circular shape or a polygonal shape. In the present embodiment, the following description discusses, as an example, the target TA that has a circular shape. However, the shape of the target TA may be changed as appropriate. In the present embodiment, the target TA has a diameter that is substantially identical to an outer diameter of an inner region of a stage body 2111 of the rotational movement stage 21 (described later), and the target TA is larger than a region that is irradiated with laser light LL.

Except for this point, the target TA of the present embodiment is configured as in the case of the target TA of Embodiment 1. That is, the target TA of the present embodiment is formed of a single layer or a plurality of layers as illustrated in Fig. 3.

Also in the ion generation device 10 in accordance with Embodiment 2, positional displacement of the target TA in a z-axis direction can occur. The ion generation device 10 in accordance with Embodiment 2 includes no holding section 14. Thus, in a case where the positional displacement correction mechanism described in Embodiment 1 is applied to Embodiment 2, it is preferable to use a movement mechanism that causes a coincidence between a position of the target TA in the z-axis direction and a position of a laser irradiation position in the z-axis direction by finely adjusting a focus of the laser light LL.

### <Movement section>

The movement section 20 includes a rotational movement stage 21 and a horizontal movement stage 22 as illustrated in (a) of Fig. 5.

The rotational movement stage 21 includes a stage 211, a cross-roller ring 212, a fastener 213, a base material 214, a motor M3, a pulley 216, and a belt 217 as illustrated in (a) and (b) of Fig. 5.

The stage 211 includes the stage body 2111 that is made of metal (stainless steel in the present embodiment) and a back plate 2112. The stage body 2111 is a cylindrical member that has a pair of bottom surfaces each of which is provided with a circular opening and a side surface which is interposed between the pair of bottom surfaces. Thus, a region of the stage body 2111 which region includes a central axis AC is provided with a through-hole (see (b) of Fig. 5).

In the stage body 2111, a vicinity of one (the negative z-axis direction side bottom surface illustrated in (a) and (b) of Fig. 5) of the pair of bottom surfaces is configured in a flange shape having a thicker side surface, as compared with a vicinity of the other (the positive z-axis direction side bottom surface illustrated in (a) and (b) of Fig. 5) of the pair of bottom surfaces. A groove 2113 is provided on an outer circumference of a flange-shaped part. The belt 217 (described later) is placed on the groove 2113. Note that the belt 217 is not illustrated in (b) of Fig. 5.

Furthermore, the inner region of the one of the pair of bottom surfaces of the stage body 2111, the inner region being ring-shaped and located further inward than the groove 2113, has a surface that is further dug down than a surface of the other region of the one of the pair of bottom surfaces. That is, a difference in level is provided at a boundary between the inner region and the other region on the one of the pair of bottom surfaces. The fastener 213 (described later) is used to fix, to the inner region, the target TA that has an outer diameter which is substantially equal to the outer diameter of the inner region and that is formed in a circular shape.

The stage 211 and the fastener 213 hold a plurality of parts of an outer edge of the target. As a result, the stage 211 and the fastener 213 function as a holding section that holds the target TA so as to (i) bring the target TA close to an induction heating section 13 and (ii) prevent occurrence of a sag in the target TA in an irradiation region irradiated with the laser light LL. The movement section 20 moves the stage 211 and the fastener 213 (holding section) in an in-plane direction of the main surface of the target TA.

As illustrated in (a) and (b) of Fig. 5, the induction heating section 13 is disposed so as to face the target TA with a gap G between the induction heating section 13 and the target TA. The target TA is classified into a part TAO that in an unheated state and a part TA1 that has been heated by the induction heating section 13.

The back plate 2112 is a circular ring-shaped plate-like member that is fixed to the other of the pair of bottom surfaces of the stage body 2111. An inner ring of the cross-roller ring 212 is fitted on or near the other of the pair of the bottom surfaces of the stage body 2111. The back plate 2112 and the stage body 2111 between which the cross-roller ring 212 is sandwiched fix the inner ring of the cross-roller ring 212 to the other of the pair of bottom surfaces.

The fastener 213 is a circular ring-shaped plate-like member that is made of metal (stainless steel in the present embodiment). The fastener 213 is configured to have an outer diameter that is slightly smaller than the outer diameter of the inner region of the stage body 2111 and an inner diameter that substantially coincides with a diameter of the through-hole of the stage 211. The fastener 213 is fitted in the inner region.

As illustrated in (b) of Fig. 5, the fastener 213 and the stage body 2111 between which the target TA is sandwiched fix the target TA to the inner region. Although not illustrated in (a) and (b) of Fig. 5, a mechanical fixing means is used to fix the fastener 213 to the stage 211. The mechanical fixing means is, for example, a plurality of bolts. Note, however, that the mechanical fixing means is not limited to the above example and can be selected as appropriate.

The base material 214 includes a base material body 2121 that is made of metal (stainless steel in the present embodiment) and a back plate 2142 (see (b) of Fig. 5). A base material body 2141 is a plate-like member contours of a pair of main surfaces of which have a shape obtained by combining a rectangular shape and a circular shape (see (a) of Fig. 5). When the main surfaces of the base material body 2141 are viewed in a plan view from the negative z-axis direction side, a region that has a contour having a circular shape is provided with an opening that is concentric with the contour. That is, a region of the base material body 2141 which region has a contour having a circular shape is circular ring-shaped. The opening has a diameter that is more than an outer diameter of the other of the pair of bottom surfaces of the stage body 2111 and slightly less than an outer diameter (diameter of an outer ring) of the cross-roller ring 212. To the opening, a part of the stage body 2111 which part includes the other of the pair of bottom surfaces is fixed via the cross-roller ring 212.

The back plate 2142 is a circular ring-shaped plate-like member that is fixed to the circular region of the base material body 2141. The outer ring of the cross-roller ring 212 is fitted in the opening of the base material body 2141. The back plate 2142 and the base material body 2141 between which the cross-roller ring 212 is sandwiched fix the outer ring of the cross-roller ring 212 to the opening of the base material body 2141.

The cross-roller ring 212 is an aspect of a roller ring and is a bearing that includes an inner ring and an outer ring which are configured to be relatively rotatable. In the movement section 20, the outer ring is fixed to the base material body 2141, and the stage body 2111 is fixed to the inner ring. The target TA is fixed to the inner region of the stage body 2111 so that the main surface of the target TA is parallel to the pair of bottom surfaces of the stage body 2111. Thus, the movement section 20 rotationally moves the target TA on the central axis AC in a plane of the main surface of the target TA (in a plane parallel to the xy plane illustrated in (a) of Fig. 5). The stage body 2111 and the fastener 213 are an example of a holding section that holds the target TA in a planar manner at a beam spot A1. The stage body 2111 and the fastener 213 between which an outer edge of the target TA is sandwiched hold a plurality of parts of the outer edge.

The pulley 216 is provided on the negative z-axis direction side main surface in a rectangular region of the base material body 2141. The pulley 216 includes a rotating shaft that is configured to be rotatable. The rotating shaft of the pulley 216 is supported by the base material body 2141.

The motor M3 is fixed to the positive z-axis direction side main surface in the rectangular region of the base material body 2141. In the present embodiment, the motor M3 is, for example, a stepping motor. The motor M3 includes a rotating shaft that is configured to be rotatable. The rotating shaft of the motor M3 is mechanically coupled to the rotating shaft of the pulley 216. Thus, rotation of the rotating shaft of the motor M3 results in rotation of the pulley 216.

The belt 217 is a circular ring-shaped member that is made of an elastic resin (rubber in the present embodiment). The belt 217 is placed on an outer edge part of the pulley 216 and the groove 2113 of the stage 211. A length of the belt 217 is determined so that moderate tension is applied in a state in which the belt 217 is placed on the outer edge part and the groove 2113. The belt 217 transmits a driving force of the motor M3 to the stage 211. Thus, in a case where the belt 217 is fed in a direction of arrows A by rotation of the pulley 216, the stage 211 rotates in a direction of an arrow B (see (a) of Fig. 5).

Note that a further pulley may be provided between the pulley 216 and the stage 211 on the negative z-axis direction side main surface of the base material body 2141. In this case, the further pulley is provided at a position that causes a path for the belt 217 to slightly meander. Moreover, the further pulley is configured so that a meandering state of the belt 217 can be adjusted. In the movement section 20, using the further pulley to adjust the meandering state makes it possible to adjust tension of the belt 217 and consequently to adjust a degree of friction between the belt 217 and each of the pulley 216 and the stage 211.

The motor M3 is controlled by the control section 17 (see (a) of Fig. 5). The control section 17 controls the motor M3 so as to rotate the stage 211 of the rotational movement stage 21 via the pulley 216 and the belt 217. In other words, the control section 17 rotationally moves the target TA in an in-plane direction of the main surface of the target TA (an in-plane direction of the plane parallel to the xy plane illustrated in (a) of Fig. 5). Thus, the stage 211, the cross-roller ring 212, the motor M3, the pulley 216, and the belt 217 of the rotational movement stage 21 are an example of a movement section. Since the control section 17 of the present embodiment need only be configured as in the case of the control section 17 of the Embodiment 1, a description thereof is omitted in the present embodiment.

### (Horizontal movement stage)

The horizontal movement stage 22 includes a base material 221 and a stage 222 as illustrated in (a) of Fig. 5. The horizontal movement stage 22 can translate, in a plane of a main surface of the base material 221 (in a plane parallel to a zx plane illustrated in (a) of Fig. 5), a position of the stage 222 that is provided upright with respect to the base material 221. That is, the horizontal movement stage 22 can move the position of the stage 222 in each of an x-axis direction and the z-axis direction. The horizontal movement stage 22 can be a precision stage that is used to assemble an optical system and that enables translation of a stage in a plane.

In the present embodiment, the position of the stage 222 is controlled by the control section 17. The control section 17 translates, in a plane, the rotational movement stage 21 that is fixed on the stage 222.

In the present embodiment, as illustrated in (a) of Fig. 5, both the horizontal movement stage 22 and the rotational movement stage 21 are driven to move the target TA in a spiral manner. Specifically, the stage 211 is rotated while the stage 222 is translated in the x-axis direction. As a result, the irradiation region irradiated with the laser light LL and the induction heating section 13 are relatively moved with respect to the target TA so as to draw a track T that is spiral and that has a center at the central axis AC.

The induction heating section 13 heats the target TAO that has been conveyed and that is in a non-heated state, so that the non-heated target TAO is made into the target TA1 that is in a heated state. The target TA1 that is in the heated state sequentially moves from the induction heating section 13 and is irradiated with the laser light LL, so that ions are generated. That is, laser-driven ions DI are substantially continuously generated. As a result, in the target TA, a region in which the laser-driven ions DI have been generated is formed as the track T that is spiral.

The present embodiment has shown, as an example, a case where the target TA is moved in a spiral manner. However, the target TA may be moved in an appropriate shape, for example, vertically and horizontally as in television scanning lines. In this case, the track T on the target TA has a scanning line shape such as television scanning lines. Alternatively, the track T can have an appropriate shape such as a straight line shape or an arc shape.

The laser light LL may be continuously or intermittently emitted. Note that, since the laser light source 15 generates a pulse laser, it is possible to consider that continuous emission of the laser light LL means, for example, emission of a pulse laser at approximately 100 Hz and that intermittent emission means, for example, emission of a pulse laser in a cycle which is sufficiently smaller than 100 Hz (e.g., a single shot).

The following description will discuss an example corresponding to Embodiment 1.

### (Example 1)

A change in temperature of the target TA during heating by the induction heating section 13 and after the heating was stopped was measured.

An induction heating coil having a shape illustrated in Fig. 2 was used as the induction heating section 13. The induction heating coil is obtained by winding a copper pipe on concentric circles three times.

A nickel tape having a thickness of 5 µm was used as the target TA, and the induction heating coil was provided in parallel to the target TA with a gap of 4 mm from the target TA.

AC power having a frequency of 400 kHz, a high frequency output of 0.6 kW, and an AC current output of 13A was supplied from the power source 131 to the induction heating coil. Cooling water was supplied from the cooler 132 into the copper pipe.

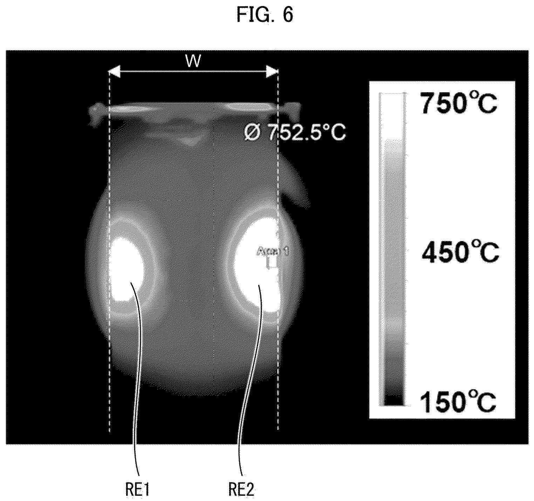

Fig. 6 is a diagram representing a temperature distribution of the target TA heated by the induction heating section 13. In the present example, the temperature distribution was measured by an infrared thermography method.

White dotted lines indicate both sides of the target TA in a width direction, and it is understood that regions RE1 and RE2 at or near the both sides have a high temperature. The regions RE1 and RE2 are regions up to approximately 5 mm inward from a side of the target TA and were heated to 600°C or higher. In contrast, a central part of the target TA between the regions RE1 and RE2 has a lower temperature than the target TA but reaches 250°C or higher. This is considered to satisfy a temperature condition required for removal of an impurity layer (water molecule layer).

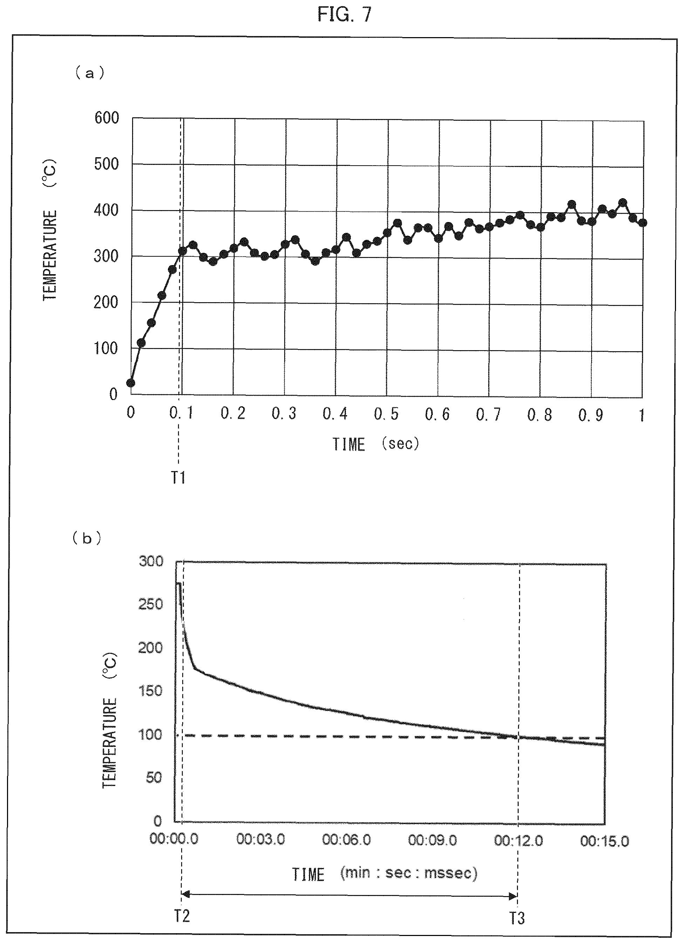

Fig. 7 provides graphs each representing a change in temperature of the target TA over time. (a) of Fig. 7 is a graph representing a change in temperature of the target TA during heating. In the present example, an induction heating output was fixed to 0.6 kW, and a change over time in temperature of the region RE2, in particular, a location that is 3 mm from a right side of the target TA is shown. In this case, a degree of vacuum was set to 0.05 Pa, which is the same as in a case where ions are actually accelerated.

As illustrated in (a) of Fig. 7, after heating was started, the temperature of the target TA reached 300°C at a time T1 of approximately 0.1 seconds and reached 400°C at a time of approximately 0.8 seconds. Thus, in the present Example, it was demonstrated that a heating rate of induction heating is very high. That is, in the ion generation device 10, the target TA can be continuously supplied to the induction heating section 13 and heated.

(b) of Fig. 7 is a graph representing a change in temperature of the target TA after completion of heating. The target TA that has been heated tends to adsorb a desorbed impurity layer again as the target TA cools. Thus, the temperature of the target TA is preferably kept at a high temperature until the target TA is irradiated with the laser light LL. As illustrated in (b) of Fig. 7, the target TA that has been heated to 600°C or higher is cooled to approximately 180°C within one second after heating is stopped at a time T2. However, thereafter, a cooling rate decreased, and it took a time (T3-T2) of approximately 12 seconds for the temperature to reach 100°C or lower.

This has resulted in finding that it is preferable to move the target TA from a heating region to a laser irradiation region within approximately 10 seconds. It is possible to reduce readhesion of the impurity layer (water molecule layer).

### (Example 2)

Example 2 shows a result of an effect of removal of an impurity layer (water molecule layer) by induction heating and an increase in efficiency of heavy ion acceleration due to the effect. The target TA that has been subjected to induction heating was irradiated with the laser light LL to generate the laser-driven ions DI.

A Ti:sapphire laser (having a center wavelength of 810 nm and a pulse width of 80 fs) was used as the laser light source 15. An off-axis parabolic mirror was used as the focusing mirror 16b to collect laser light up to a diameter of 2 µm to 3 µm on the target TA. In this case, energy per pulse of the laser light LL was approximately 500 mJ on the target TA film.

A nickel tape having a thickness of 5 µm was used as the target TA, and the target TA was continuously moved by being wound by the pulley 122.

The laser-driven ions DI were separated by a Thomson parabola ion analyzer in accordance with a charge-to-mass ratio, and an energy distribution was measured.

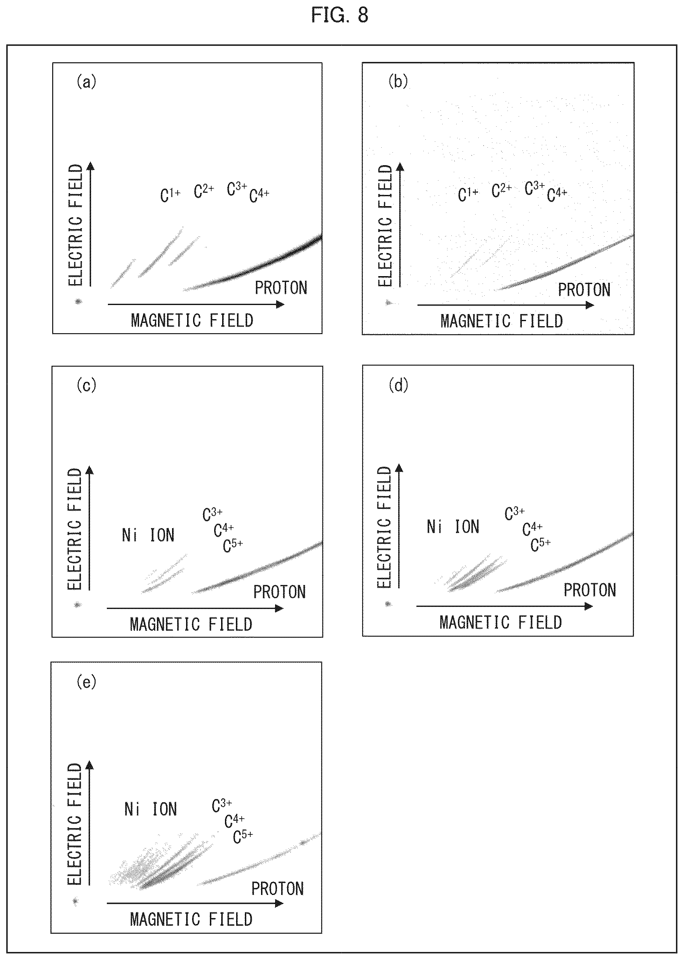

Fig. 8 shows a result of analysis of ions emitted from the target TA consisting of a single layer of nickel. (a) of Fig. 8 shows a result in a case where the laser light LL was emitted without induction heating, and (b) to (e) of Fig. 8 show results in a case where the laser light LL was emitted 20 seconds, 10 seconds, 5 seconds, and 2.4 seconds after induction heating at 0.6 kW.

In the case of non-heating, hydrogen ions (protons) originating from an impurity layer (water molecule layer) were dominantly observed. Furthermore, monovalent to tetravalent carbon ions were observed.

Differences were found over time in the case of heating. In the case of 20 seconds after heating, ions similar to those in the case of non-heating were observed. It is considered that an impurity layer containing hydrogen was adsorbed on the target TA in 20 seconds after heating.

In the case of 10 seconds and 5 seconds after heating, hydrogen ions (protons) were still dominant, but the maximum valence number of carbon ions increased to five. Furthermore, carbon ions in a high energy region increased. Nickel ions were also observed. This indicates that removal of the impurity layer (water molecule layer) by heating has resulted in acceleration of carbon ions by a higher intensity sheath electric field.

In contrast, in the case of 2.4 seconds after heating, hydrogen ions (protons) decreased and carbon ions became most dominant.

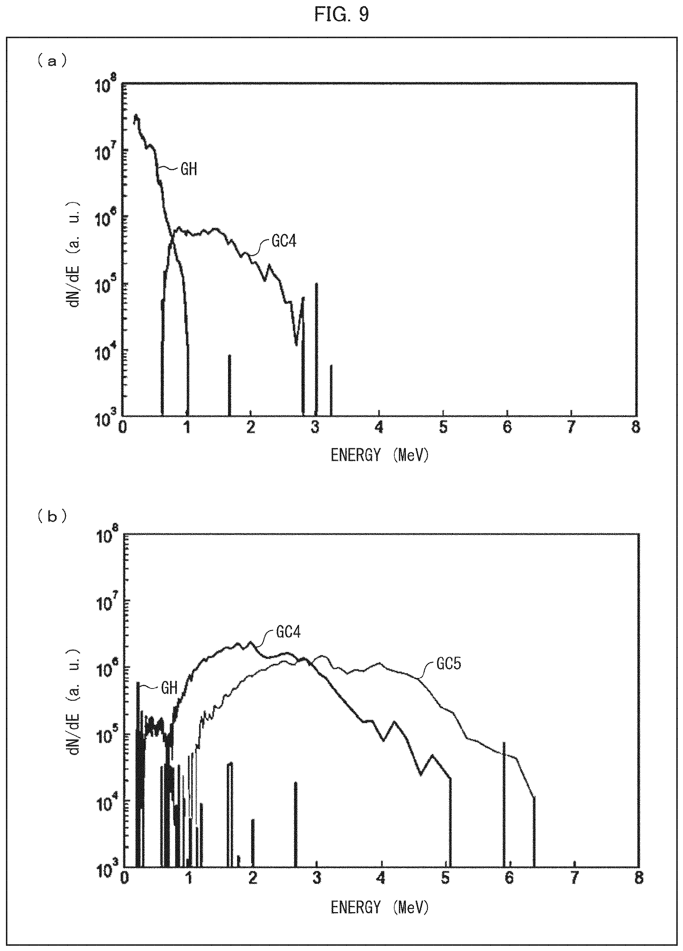

Fig. 9 provides graphs representing energy spectra of ions emitted from the target TA consisting of a single layer of nickel. (a) and (b) of Fig. 9 represent energy spectra of hydrogen ions (protons) and carbon ions for the case of non-heating and the case of heating (after 2.4 seconds).

Maximum energy of hydrogen ions (protons) was 1 MeV, which did not change depending on the presence or absence of heating. However, heating has significantly reduced the amount of hydrogen ions (protons) with energy of 0.5 MeV or less to approximately one one-hundredth.

Tetravalent carbon ions were accelerated up to 3 MeV in the case of non-heating, whereas pentavalent carbon ions were accelerated up to 6.5 MeV in the case of heating. That is, both the maximum valence number and maximum energy of carbon ions increased. It is considered that removal of a surface polluted layer by induction heating minimized acceleration of hydrogen ions (protons), so that acceleration of carbon ions was caused with high efficiency.

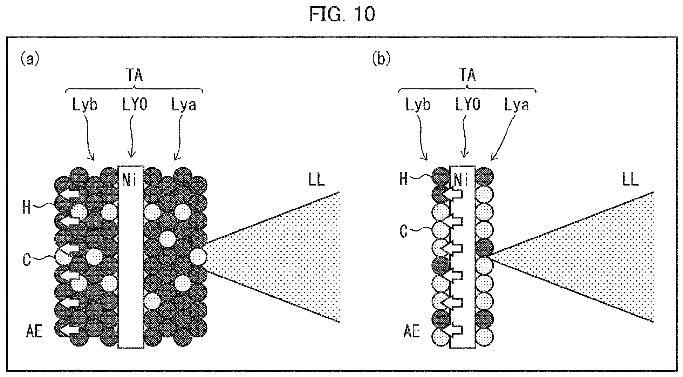

Fig. 10 is a diagram schematically illustrating an impurity layer having adhered to a structure of the target TA which structure is assumed from a result of detection of ions in Example 2. (a) and (b) of Fig. 10 represent structures of the target TA in the case of non-heating, the case of 20 seconds or more after heating, and the case of 10 seconds or less after heating.

As described earlier, hydrogen ions (protons) and carbon ions were observed in the case of non-heating and the case of 20 seconds or more after heating. In this case, as illustrated in (a) of Fig. 10, impurity layers LYa and Lyb that are thick and that are made of, for example, water molecules and alkane (oil) have adhered on both surfaces of a nickel layer LYO. As a result, a region in which an acceleration electric field for accelerating ions is generated is considered not to reach the target TA.

In contrast, in the case of 10 seconds or less after heating, a ratio of the number of hydrogen ions (protons) to the number of carbon ions was improved, and acceleration of nickel ions was further observed. In this case, as illustrated in (b) of Fig. 10, it is considered that the impurity layers LYa and LYb were made thin by heating, and the acceleration electric field has reached inside a nickel film.

### (Example 3)

In Example 3, the target TA as illustrated in (b) of Fig. 3 was used. The target TA consists of two layers, which are a nickel layer LYO and a carbon layer LY1 formed on the nickel layer LYO. Thus, as compared with Example 2, only the target TA was changed, and the other conditions were similar.

The target TA was obtained by using vapor deposition to form, on a nickel layer having a thickness of 5 µm, a 100 nm carbon layer as an ion species. The target TA was continuously moved by being wound by the pulley 112.

The target TA moving was heated by the induction heating section 13 to remove a polluted layer having adhered to a surface of the target TA. Thereafter, the surface of the target TA was irradiated with the laser light LL to generate and accelerate carbon ions.

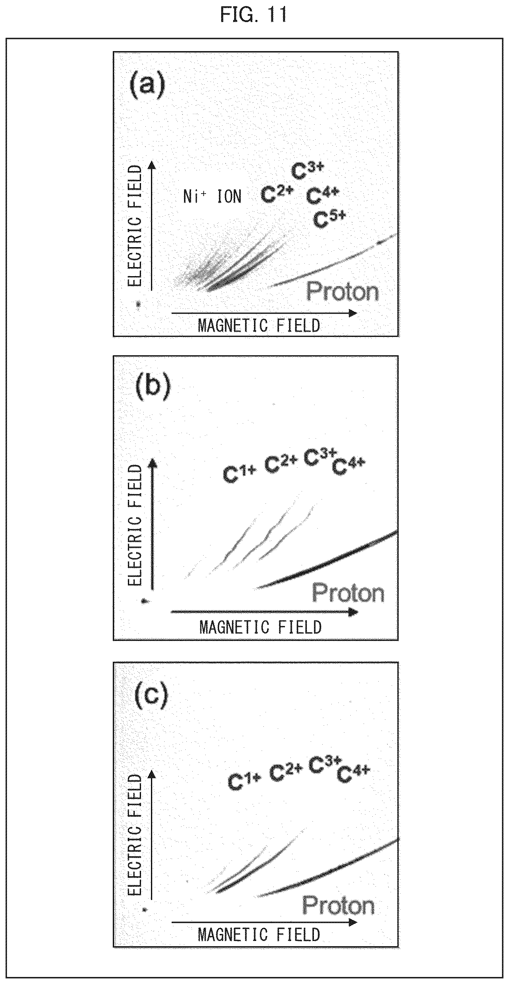

Fig. 11 shows a result of analysis of ions emitted from the target TA consisting of two layers of nickel and carbon. (a) of Fig. 11 shows a result of analysis of a heated target consisting of a single layer of nickel, with another reference to (e) of Fig. 8 as a comparative example. (b) and (c) of Fig. 11 represent results of analysis of the non-heated target TA consisting of two layers and the heated target TA consisting of two layers.

As illustrated in (a) of Fig. 11, in a case of the heated target TA consisting of a single layer of nickel, nickel ions are mixed in carbon ion beams. That is, carbon ions and nickel ions are mixed with relatively heavy particles. This is not preferable in some applications. For example, in heavy ion cancer therapy, the depth of the Bragg peak in the human body varies depending on an ion species. Thus, mixing of an ion species different from an ion species (e.g., carbon ions) used in the therapy should be avoided. Note that hydrogen ions (protons) greatly differ in mass-to-charge ratio, and thus can be easily removed.

In contrast, as illustrated in (b) and (c) of Fig. 11, no nickel ions were observed in a case where the target TA consisting of multilayers of nickel and carbon was used.

In the case of (b) of Fig. 11, it is considered that the target TA, which was in a non-heated state, made an impurity layer thick and prevented an acceleration electric field from entering into the nickel layer. However, in case of (c) of Fig. 11, although the target TA was heated and the impurity layer was made thin, no nickel ions were observed.

This is considered to be because the carbon layer LY1 having been vapor-deposited with a thickness of 100 nm prevented entry of the acceleration electric field into the nickel layer even after heating made the impurity layers (polluted moisture layers) LYa and LYb thin. Thus, forming the layer LY1 of an ion species on a back surface of the conductor layer LYO makes it possible to ionize an ion species constituting the layer LY1, without ionizing atoms constituting the conductor layer.

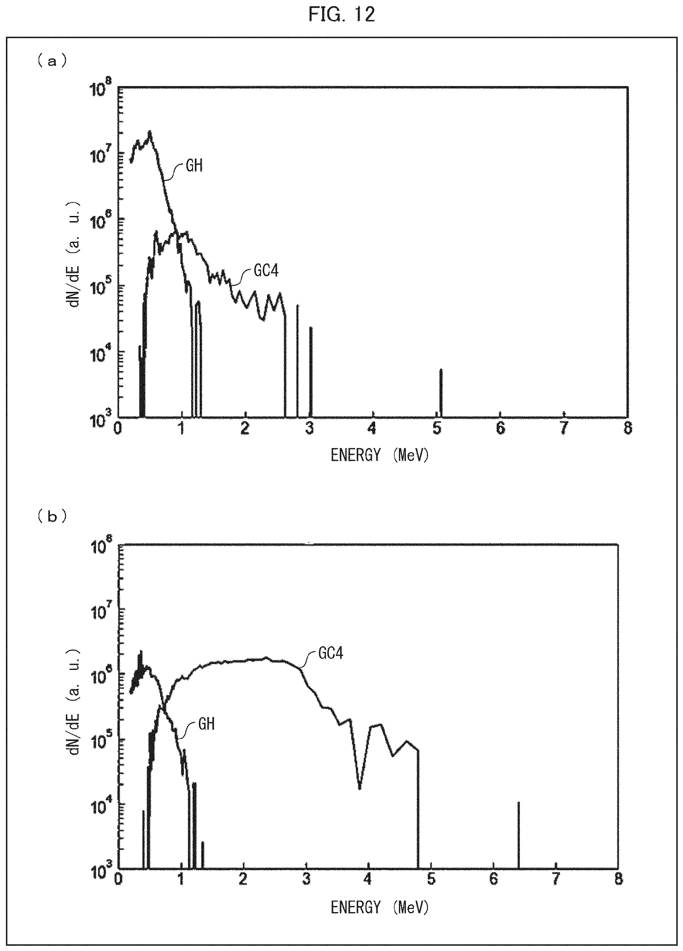

Fig. 12 provides graphs representing energy spectra of ions emitted from the target TA consisting of two layers of nickel and carbon. (a) of Fig. 12 corresponds to the non-heated target TA, and (b) of Fig. 12 corresponds to the target TA at 2.4 seconds after heating.

Maximum energy of hydrogen ions (protons) was 1 MeV, which did not change depending on the presence or absence of heating. However, heating reduced the number of hydrogen ions (protons) to one tenth.

In contrast, tetravalent carbon ions were accelerated to 3 MeV in the case of non-heating, whereas the tetravalent carbon ions were accelerated to 4.8 MeV in the case of heating. That is, maximum energy increased. It is considered that removal of a surface polluted layer by induction heating minimized acceleration of hydrogen ions (protons), so that carbon ions were accelerated with higher efficiency.

### (Example 4)

In Example 4, the target TA in which heavy ions were embedded by ion sputtering was used. The target TA had a multilayer structure consisting of a conductor layer for induction heating and a layer of an ion species. The layer of the ion species may be composed of not only an atom or molecule constituting a solid, but also, for example, a gas molecule (helium, argon, oxygen, or neon) that is not solid at normal temperature.

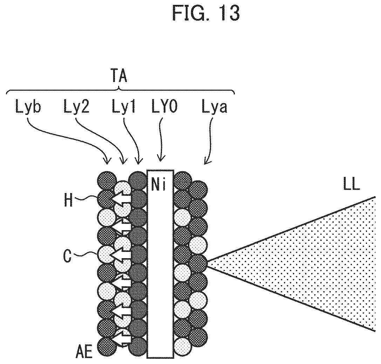

In Example 4, an argon layer Ly1 was formed by sputtering argon on a surface of the nickel layer LYO having a thickness of 5 µm, and then a carbon layer Ly2 of 100 nm was vapor-deposited. This results in formation of the target TA that has a three-layer structure including the nickel layer (conductor layer) LYO, the argon layer Ly1, and the carbon layer Ly2. In a case where the target TA is placed in the atmosphere, the impurity layers Lya and Lyb are formed, so that a layer structure as illustrated in Fig. 13 is obtained.

The target TA was heated by induction heating to remove the impurity layers Lya and Lyb having adhered to a surface of the target TA. Thereafter, the target TA was irradiated with the laser light LL to accelerate ions.

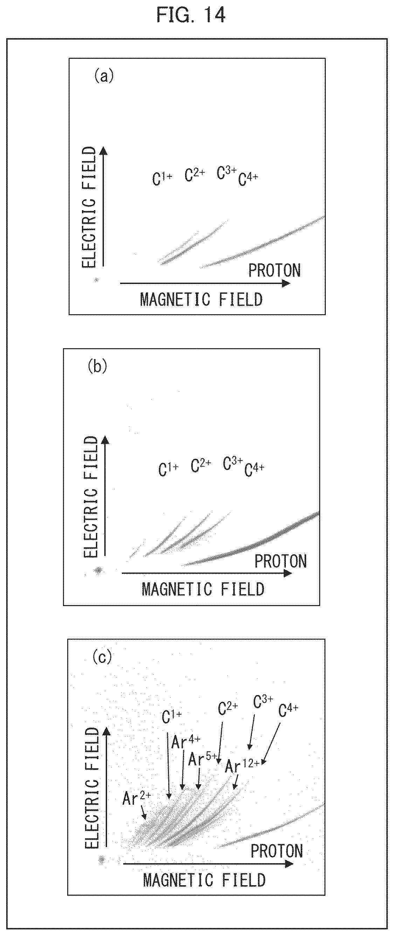

Fig. 14 provides graphs representing ions emitted from the target TA consisting of three layers of nickel, argon, and carbon. (a) of Fig. 14 shows a result of analysis of the heated target TA consisting of two layers of nickel and carbon, with another reference to (c) of Fig. 11 for comparison. (b) and (c) of Fig. 14 represent results of analysis of the non-heated and heated target TA consisting of three layers of nickel, argon, and carbon.

As illustrated in (b) of Fig. 14, hydrogen ions (protons) derived from a surface polluted layer and carbon ions were detected in the case of non-heating. In contrast, as illustrated in (c) of Fig. 14, argon ions embedded in the nickel layer by sputtering were detected in addition to the hydrogen ions (protons) and the carbon ions in the case of heating. That is, ions that are gaseous at room temperature can be embedded in a conductor layer with use of sputtering.

Aspects of the present invention can also be expressed as follows:
An ion generation device in accordance with Aspect 1 of the present invention includes: an induction heating section (13) that carries out induction heating with respect to a target (TA) which includes a conductor layer and which is in film form; and a laser irradiation section (laser light source 15, focusing mirror 16b) that irradiates the target with laser light so as to generate ions from the target, the target having been subjected to induction heating. A dielectric heating section is capable of heating a target without contacting the target. Thus, as in the case of using a resistance heating method, it is possible to remove an impurity layer from the target without forming a pair of electrodes in the target. This makes it possible to move a position of the target while heating the target. Thus, it is possible to, while removing an impurity layer which can be formed on a surface of the target, sequentially supply the target to an irradiation region irradiated with laser light. Note here that the above-described concept that it is possible to sequentially supply the target includes a concept that it is also possible to supply continuous targets.

In Aspect 2 of the present invention, an ion generation device is configured such that, in Aspect 1, the induction heating section has an induction heating coil that is close to the target. This allows the target to be rapidly heated by the induction heating coil.

In Aspect 3 of the present invention, an ion generation device is configured to, in Aspect 1 or 2, further include the target. Thus, the target can be included in one of components of the present ion generation device.

In Aspect 4 of the present invention, an ion generation device is configured such that, in any one of Aspects 1 to 3, the target includes the conductor layer and a first ion source layer that is provided on at least one main surface of the conductor layer and that is composed of a first ion species which is ionized by being irradiated with the laser light. With this, a first ion source layer that is composed of even a first ion species which is not electrically conductive makes it possible to generate ions of the first ion species while removing an impurity layer. Further, even in a case where the first ion species is an ion species that is difficult to form in layer form, it is possible to generate ions of the first ion species. Note that the first ion species may be composed of a combination of a plurality of ion species.

In Aspect 5 of the present invention, an ion generation device is configured such that, in Aspect 4, the target further includes a second ion source layer that contains a second ion species which is ionized by being irradiated with the laser light. This makes it possible to generate ions of the first and second ion species. Note that the second ion species may be composed of a combination of a plurality of ion species.

In Aspect 6 of the present invention, an ion generation device is configured to, in any one of Aspects 1 to 5, further include a holding section that holds the target so as to (i) bring the target close to the induction heating section and (ii) prevent occurrence of a sag in the target in an irradiation region irradiated with the laser light. This allows a target whose shape is retained to be efficiently subjected to induction heating and laser light irradiation.

In Aspect 7 of the present invention, an ion generation device is configured to, in Aspect 6, further include a movement section that moves a position of the target relative to the irradiation region. This makes it possible to move the relative position of the target and continuously generate ions.

In Aspect 8 of the present invention, an ion generation device is configured such that, in Aspect 7, the holding section has a first holding section that holds the target so as to bring the target close to the induction heating section and a second holding section that holds the target in the irradiation region, the first holding section and the second holding section are spaced apart from each other when the irradiation region is viewed in a plan view, and the movement section moves the target from the first holding section to the second holding section. This makes it possible to independently provide a first holding section and a second holding section to an induction heating section and an irradiation region, so that a holding section is easily designed.

In Aspect 9 of the present invention, an ion generation device is configured such that, in any one of Aspects 1 to 7, when an irradiation region irradiated with the laser light is viewed in a plan view, the irradiation region is included in a region of the target which region is close to the induction heating section. According to this, by irradiating a target with laser light while heating the target by dielectric heating, it is possible to prevent readsorption of impurities onto the target that has been heated and has not been irradiated with the laser light. This makes it possible to generate desired ions with higher efficiency and higher purity.

In Aspect 10 of the present invention, an ion generation device is configured such that, in any one of Aspects 7 to 9, the target is in tape form, the movement section has a first pulley through which the target is fed and a second pulley around which the target is wound, and the holding section is disposed between the first pulley and the second pulley. Using the target in tape form makes it easy to continuously generate ions.

In Aspect 11 of the present invention, an ion generation device is configured such that, in any one of Aspects 7 to 9, the target has a circular or polygonal shape, the holding section holds a plurality of parts of an outer edge of the target, and the movement section moves the holding section in an in-plane direction of a main surface of the target. Using the target having a circular or polygonal shape makes it possible to continuously generate ions.

An ion generation method in accordance with Aspect 12 of the present invention includes: the step of carrying out induction heating with respect to a target that includes a conductor layer and that is in film form; and the step of irradiating the target with laser light so as to generate ions from the target, the target having been subjected to induction heating. This makes it possible to move a position of the target while heating the target. Thus, it is possible to, while removing an impurity layer which can be formed on a surface of the target, sequentially supply the target to an irradiation region irradiated with laser light.

In Aspect 13 of the present invention, an ion generation method is configured to, in Aspect 12, further include the step of moving a position of the target relative to an irradiation region irradiated with the laser light. This makes it easy to rapidly heat the target and continuously generate ions.

A target for ion generation in accordance with Aspect 14 of the present invention includes: a conductor layer; and a first ion source layer that is provided on at least one main surface of the conductor layer and that is composed of a first ion species which is ionized by being irradiated with laser light. With this, a first ion source layer that is composed of even a first ion species which is not electrically conductive makes it possible to carry out heating by induction heating to remove an impurity layer. Note that the first ion species may be composed of a combination of a plurality of ion species.

In Aspect 15 of the present invention, a target for ion generation is configured to, in Aspect 14, further include a second ion source layer that contains a second ion species which is ionized by being irradiated with the laser light. This makes it possible to generate ions of the first and second ion species. Note that the second ion species may be composed of a combination of a plurality of ion species.

### [Additional remarks]

The present invention is not limited to the embodiments, but can be altered by a skilled person in the art within the scope of the claims. The present invention also encompasses, in its technical scope, any embodiment derived by combining technical means disclosed in differing embodiments.

### Reference Signs List

10 Ion generation device
11 Chamber
111 Port
12, 20 Movement section
13 Induction heating section
131 Power source
132 Cooler
14 Holding section
15 Laser light source
16a Pulse compressor
16b Focusing mirror
17 Control section

## Claims

1. An ion generation device comprising:
an induction heating section that carries out induction heating with respect to a target which includes a conductor layer and which is in film form; and
a laser irradiation section that irradiates the target with laser light so as to generate ions from the target, the target having been subjected to induction heating.

2. The ion generation device as set forth in claim 1, wherein
the induction heating section has an induction heating coil.

3. The ion generation device as set forth in claim 1 or 2,
further comprising the target.

4. The ion generation device as set forth in claim 1, wherein
the target includes
the conductor layer and
a first ion source layer that is provided on at least one main surface of the conductor layer and that is composed of a first ion species which is ionized by being irradiated with the laser light.

5. The ion generation device as set forth in claim 4, wherein
the target further includes a second ion source layer that contains a second ion species which is ionized by being irradiated with the laser light.

6. The ion generation device as set forth in claim 1,
further comprising a holding section that holds the target so as to (i) bring the target close to the induction heating section and (ii) prevent occurrence of a sag in the target in an irradiation region irradiated with the laser light.

7. The ion generation device as set forth in claim 6,
further comprising a movement section that moves a position of the target relative to the irradiation region.

8. The ion generation device as set forth in claim 7, wherein
the holding section has
a first holding section that holds the target so as to bring the target close to the induction heating section and
a second holding section that holds the target in the irradiation region,
the first holding section and the second holding section are spaced apart from each other when the irradiation region is viewed in a plan view, and
the movement section moves the target from the first holding section to the second holding section.

9. The ion generation device as set forth in claim 1, wherein
when an irradiation region irradiated with the laser light is viewed in a plan view, the irradiation region is included in a region of the target which region is close to the induction heating section.

10. The ion generation device as set forth in claim 7, wherein
the target is in tape form,
the movement section has a first pulley through which the target is fed and a second pulley around which the target is wound, and
the holding section is disposed between the first pulley and the second pulley.

11. The ion generation device as set forth in claim 7, wherein
the target has a circular or polygonal shape,
the holding section holds a plurality of parts of an outer edge of the target, and
the movement section moves the holding section in an in-plane direction of a main surface of the target.

12. An ion generation method comprising:
the step of carrying out induction heating with respect to a target that includes a conductor layer and that is in film form; and
the step of irradiating the target with laser light so as to generate ions from the target, the target having been subjected to induction heating.

13. The ion generation method as set forth in claim 12,
further comprising the step of moving a position of the target relative to an irradiation region irradiated with the laser light.

14. A target for ion generation, comprising:
a conductor layer; and
a first ion source layer that is provided on at least one main surface of the conductor layer and that is composed of a first ion species which is ionized by being irradiated with laser light.

15. The target for ion generation as set forth in claim 14,
further comprising a second ion source layer that contains a second ion species which is ionized by being irradiated with the laser light.
